Europäisches Patentamt

European Patent Office

Office européen des brevets

(11) Publication number: **0 064 354**
**B1**

(12) EUROPEAN PATENT SPECIFICATION

(45) Date of publication of patent specification: **06.11.85**

(21) Application number: **82302018.5**

(22) Date of filing: **20.04.82**

(51) Int. Cl.⁴: **C 12 N 1/20, C 12 P 19/04,**
**E 21 B 43/22, C 09 K 7/02,**
**A 01 N 25/04, A 23 L 1/04 //**
**C12R1/05, C12P19/26**

(54) Heteropolysaccharide S-198.

(30) Priority: **23.04.81 US 256628**
**23.04.81 US 256627**
**23.04.81 US 256751**
**23.04.81 US 256749**

(43) Date of publication of application:
**10.11.82 Bulletin 82/45**

(45) Publication of the grant of the patent:
**06.11.85 Bulletin 85/45**

(84) Designated Contracting States:
**AT BE CH DE FR GB IT LI LU NL SE**

(56) References cited:
**FR-A-2 190 377**
**FR-A-2 202 939**
**GB-A-2 058 107**
**US-A-3 856 626**

**CHEMICAL ABSTRACTS, vol. 89, no. 13, 25th**
**September 1978, page 404, no. 103458x,**
**Columbus, Ohio, USA M. YAMAGUCHI et al.:**
**"Microbial production of polysaccharide. II.**
**Production of viscous polysaccharide from**
**ethylene glycol"**

(73) Proprietor: **MERCK & CO. INC.**
**126, East Lincoln Avenue P.O. Box 2000**
**Rahway New Jersey 07065 (US)**

(72) Inventor: **Peik, Jerry A.**
**11291 Tribuna Avenue**
**San Diego California 92131 (US)**
Inventor: **Steenbergen, Suzanna M.**
**15216 El Monte Road**
**Lakeside California 92040 (US)**
Inventor: **Hayden, Harold R.**
**1805 Idaho Avenue**
**Escondido California 92027 (US)**
Inventor: **Shim, Jaewon L.**
**6079 Via Regla**
**San Diego California 92122 (US)**
Inventor: **Chang, Joe H-S**
**3027 Garboso Street**
**Carlsbad California 92008 (US)**
Inventor: **Pickens, Patrick A.**
**4567 Cleveland Avenue No. 3**
**San Diego California 92116 (US)**
Inventor: **Colegrove, George T.**
**5238 Fontaine Street**
**San Diego California 92120 (US)**

Courier Press, Leamington Spa, England.

(56) References cited:
**ADVANCES IN CARBOHYDRATE CHEMISTRY AND BIOCHEMISTRY, vol. 36, 1979, Academic Press, pages 265-313, New York, USA P.A. SANDFORD: "Exocellular, microbial polysaccharides"**

(74) Representative: **Crampton, Keith John Allen et al**
**D YOUNG & CO 10 Staple Inn**
**London WC1V 7RD (GB)**

# 0 064 354

**Description**

It is known that heteropolysaccharides can be produced by certain microorganisms. Some of these heteropolysaccharides function as hydrophilic colloids and because of their viscosity and rheology properties have been used as thickening agents for aqueous systems.

As with other fields of technology, research has continued with the objective of discovering new heteropolysaccharides having useful properties as thickening, suspending and/or stabilizing agents.

The present invention provides a novel heteropolysaccharide, herein designated Heteropolysaccharide S-198, and a method for producing it by bacterial fermentation, comprising growing the organism ATCC 31853 in an aqueous nutrient medium by the submerged aerobic fermentation of an assimilable carbohydrate source and recovering the heteropolysaccharide S-198. The microorganism is described in more detail below.

Heteropolysaccharide S-198 is a high-molecular-weight polysaccharide containing primarily carbohydrate. It is sometimes referred to as a "gum" but it is believed that the heteropolysaccharide terminology is more accurate and precise. In the following description, it will sometimes be referred to simply as S-198.

S-198 has been found to possess the unusual property of high viscosity retention even after high shear and/or heat treatment. This property is valuable for a number of industrial applications, such as high-water-based lubricating fluids (HWBF), and various oil-field applications such as circulating drilling fluids, fracturing or acidizing fluids, or workover and completion fluids, and as a thickener and suspending agent in flowable pesticides or other agricultural suspensions.

For instance, a solution of 0.28% of biogum S-198 (a blend of three different fermentation batches in equal amounts) had an initial viscosity of 193 (LBT Brookfield, rm. temp., sp. No. 2,60 rpm). Each of several samples was passed through a piston-type, air operated, boost pump: 6 passes at 2000 psi (13.8 MPa), and 1 pass at each of 3000 psi (30.7 MPa), 4000 psi, (27.6 MPa) and 5000 psi (34.5 MPa). Representative viscosity results are as indicated, which show good shear stability.

| | 2000 psi (13.8 MPa) | | | | | |
| | 1 | 2 | 3 | 4 | 5 | 6 |
| --- | --- | --- | --- | --- | --- | --- |
| Visc. | 208 | 232 | 222 | 215 | 198 | 193 |

| | 3000 psi (20.7 MPa) | 4000 psi (27.6 MPa) | 5000 psi (34.5 MPa) |
| --- | --- | --- | --- |
| Visc. | 235 | 229 | 222 |

A typical HWBF composition has 90—95% water, a shear stable viscosity imparting amount of S-198 gum (0.1—2%), and about 3—9% of one or more additives which are wetting agents, defoamers, corrosion inhibitors, anti-wear agents, and biological control agents. Optionally, up to 33% anti-freeze may be added.

S-198 can be used at 0.07—1% in well-treating fluids, by which is meant a variety of compositions such as circulating drilling fluids, workover and completion fluids and stimulation fluids (such as hydraulic fracturing and acidizing fluids). Typical fluid fermentations are:

| | | | |
| --- | --- | --- | --- |
| 1. | Seawater | 1 bbl | (159 litres) |
| | S-198 | 0.25 to 3.5 lb | (113 g to 1.6 Kg) |
| | NaOH | to *ca.* pH 9 | |
| | Starch (optional) | 1 lb | (454 g) |
| | | | |
| 2. | Fresh Water | 1 bbl | (159 litres) |
| | S-198 | 0.25 to 3.5 lb | (113 g to 1.6 Kg) |
| | KCl | 3.5 to 17.5 lb | (1.6 to 7.9 Kg) |
| | Bentonite | 2 to 20 lb | (900 g to 9 Kg) |
| | NaOH | to *ca.* pH 9 | |
| | Carboxymethylcellulose (optional) | ½ lb | (226 g) |
| | | | |
| 3. | Aq. HCl (1—15 wt.%) 99% | | |
| | Corrosion inhibitor | 0.05% | |
| | S-198 | 0.1—1.0% | |

"Flowable pesticides" means aqueous suspensions of insoluble and often hydrophobic materials used as insecticides, herbicides, fungicides, miticides, and/or aquaherbicides. These products typically require extensive milling to intimately mix the components into concentrated suspensions, which are then diluted with water prior to use. A typical concentrate formulation is:

3

**0 064 354**

|  | Wt. % |
|---|---|
| Water | 30—60 |
| Antifreeze | 3—10 |
| Surfactant | 1—3 |
| Dispersant | 1—4 |
| Antifoam | 0.25—1 |
| Active material | 10—60 |
| S-198 | 0.08—0.25 |

As mentioned above Heteropolysaccharide S-198 may be (and in accordance with the present invention is) prepared by fermentation of a suitable nutrient medium with an organism, an *Alcaligenes* species. An unrestricted permanent deposit of an organism of this species used in making the heteropolysaccharide was made with the American Type Culture Collection on 31 March 1981 under Accession No. ATCC 31853. A biologically pure culture of ATCC 31853 constitutes another embodiment of the present invention.

The organism was isolated from a pond water sample obtained in the Rocky Mountains near Denver, Colorado. The organism was picked as a gummy colony after five days' incubation at 30°C from a YM agar plate. The isolate was then pure cultured on nutrient agar.

Taxonomic studies of ATCC 31853 indicate that it does not correspond to any of the *Alcaligenes* species in Bergey's Manual.

A YM flask seed was started with a fresh NA plate and placed on a gyrotary shaker at 30°C. Approximately 24 hours later this seed was used to inoculate an E-1 flask with 3% hydrolysed starch as the carbon source. The flask was also placed on a shaker at 30°C. Approximately 72 hours later the flask was noted to have viscous beer and upon addition of two volumes of 99% IPA a fibrous precipitate was observed.

Another YM seed flask was prepared in the above fashion and used at 24 hours to inoculate five flasks containing various media and 3% glucose. These flasks were incubated on a shaker at 30°C for about 72 hours at which the pH, viscosity, gum yield, and product viscosity were measured. The results are shown in Table 1.

E-1 medium contains 5 gms of dipotassium phosphate, 0.1 gm of magnesium sulphate, 0.9 gm of ammonium nitrate, 0.5 gm of the enzymatic digest of soybean meal sold by Central Soya Chemurgy Division under the registered Trade mark "Promosoy 100", 30 gms of dextrose and 1 liter of tap water. The pH of the E-1 medium is about 7.6 to 7.8.

4

TABLE 1
Effect of media on gum production

| Medium | pH | Beer Vis. (mPa · s) | Gum Yield (%) | 1% Product Vis. (mPa · s) |
|---|---|---|---|---|
| E-1 | 6.3 | 4800 | 1.144 | 1950 |
| E-1−NH$_4$NO$_3$+0.19% NaNO$_3$ | 6.7 | 2050 | 0.976 | ND[1] |
| E-1+0.15% Promosoy® | 5.4 | 5400 | 1.602 | 1900 |
| E-1+HoLe[2] salts | 6.3 | 3000 | 0.934 | ND |

[1] ND: Not determined

[2] HoLe salts: An aqueous solution comprising

| | Conc. in stock soln. (mg/L) | Conc. in final medium (ppm) |
|---|---|---|
| H$_3$BO$_3$ | 285.0 | 0.05 B$^{+3}$ |
| MnCl$_2$4H$_2$O | 1800 | 0.5 Mn$^{+2}$ |
| FeSO$_4$ | 1360 | 0.5 Fe$^{+2}$ |
| Na$_2$C$_4$H$_4$O$_6$ · 2H$_2$O (Na Tartrate) | 2098 | |
| CuCl$_2$ | 26.9 | 0.01 Cu$^{+2}$ |
| ZnCl$_2$ | 20.8 | 0.02 Zn$^{+2}$ |
| CoCl$_2$ · 6H$_2$O | 73.99 | 0.01 Co$^{+2}$ |
| Na$_2$MoO$_4$ · 2H$_2$O | 25.2 | 0.01 Mo$^{+6}$ |

Fermentation conditions

Heteropolysaccharide S-198 is produced during the aerobic fermentation of suitable aqueous nutrient media under controlled conditions via inoculation with the organism ATCC 31853. The media contain a source of carbon, nitrogen, and inorganic salts.

In general, carbohydrates (for example, glucose, fructose, maltose, sucrose, xylose, mannitol and the like) can be used either alone or in combination as sources of assimilable carbon in the nutrient medium. The exact quantity of the carbohydrate source or sources utilized in the medium depends in part upon the other ingredients of the medium but, in general, the amount of carbohydrate usually varies between about 2% and 5% by weight of the medium. These carbon sources may be combined in the medium. In general, many proteinaceous materials may be used as nitrogen sources in the fermentation process. Suitable nitrogen sources include, for example, yeast hydrolysates, primary yeast, soybean meal, cottonseed flour, hydrolysates of casein, cornsteep liquor, distiller's solubles or tomato paste and the like. The sources of nitrogen, either alone or in combination, are used in amounts preferably ranging from about 0.05% to 0.2% by weight of the aqueous medium. Promosoy 100® can be used in the range 0.005 to 0.4%.

Among the nutrient inorganic salts which can be incorporated in the culture media are the customary salts capable of yielding sodium, potassium, ammonium, calcium, phosphate, sulfate, chloride, carbonate, and the like ions. Also included are trace metals such as cobalt, manganese, iron and magnesium.

It should be noted that the media described in the examples are merely illustrative of the wide variety of media which may be employed, and are not intended to be limiting.

As an alternate medium, S-198 may be given under low Ca$^{++}$ conditions, i.e., in deionized water or some other aqueous system substantially free of Ca$^{++}$ ions (i.e., less than about 4 ppm Ca$^{++}$ per 1% gum in the final fermentor broth).

The fermentation is carried out at temperatures ranging from about 25°C to 35°C; however, for optimum results it is preferable to conduct the fermentation at temperatures of from about 28°C to 32°C. The pH of the nutrient media for growing the ATCC 31853 culture and producing the polysaccharide S-198 can vary from about 6 to 8.

Although the polysaccharide S-198 is produced by both surface and submerged culture, it is preferred to carry out the fermentation in the submerged state.

A small scale fermentation is conveniently carried out by inoculating a suitable nutrient medium with

5

the culture and, after transfer to a production medium, permitting the fermentation to proceed at a constant temperature of about 30°C on a shaker for several days.

The fermentation is initiated in a sterilized flask of medium via one or more stages of seed development. The nutrient medium for the seed stage may be any suitable combination of carbon and nitrogen sources. The seed flask is shaken in a constant temperature chamber at about 30°C for 1—2 days, or until growth is satisfactory, and some of the resulting growth is used to inoculate either a second stage seed or the production medium. Intermediate stage seed flasks, when used, are developed in essentially the same manner; that is, part of the contents of the flask from the last seed stage are used to inoculate the production medium. The inoculated flasks are shaken at a constant temperature for several days, and at the end of the incubation period the contents of the flasks are recovered by precipitation with a suitable alcohol such as isopropanol, conveniently in the form of CBM (an 85:15 alcohol:water constant boiling mixture).

For large scale work, it is preferable to conduct the fermentation in suitable tanks provided with an agitator and a means of aerating the fermentation medium. According to this method, the nutrient medium is made up in the tank and sterilized by heating at temperatures of up to about 121°C. Upon cooling, the sterilized medium is inoculated with a previously grown seed of the producing culture, and the fermentation is permitted to proceed for a period of time as, for example, from 2 to 4 days while agitating and/or aerating the nutrient medium and maintaining the temperature at about 30°C. This method of producing the S-198 is particularly suited for the preparation of large quantities.

Although ATCC 31853 can be grown under a broad spectrum of media conditions, the following preferred conditions are recommended.

1. Culture maintenance

The culture grows quite well on nutrient agar (NA) or YM agar, but NA is preferred for culture maintenance.

2. Seed preparation

Seed preparation for this organism is started in 500 ml flasks containing 100 ml YM medium incubated at 30°C. The YM seeds are then used at 24—30 hrs to inoculate a 5L fermentor containing 3L of the seed medium. The composition of the seed medium is as follows:

| | |
|---|---|
| 3.0% | Glucose |
| 0.5% | $K_2HPO_4$ |
| 0.06% | Promosoy 100 |
| 0.12% | $NH_4NO_3$ |
| 0.01% | $MgSO_4 \cdot 7H_2O$ |
| 0.1 ml/L | HoLe Salts |
| 2 ml | antifoam additive |

A 5 to 10% inoculum size is used at 48 hrs to inoculate the final fermentor, containing 50L of medium.

3. 70L Fermentor medium

| | |
|---|---|
| 3.0% | Glucose |
| 0.05% | $K_2HPO_4$ |
| 0.06% | Promosoy 100® |
| 0.12% | $NH_4NO_3$ |
| 0.01% | $MgSO_4 \cdot 7H_2O$ |
| 0.1 m/L | HoLe Salts |
| 20 ml | antifoam additive |

The pH is controlled at 6.5—7.2, preferably 6.8; the temperature at 30°C. Aeration is 20L/min. Agitation is employed starting at 300 rpm increased to 600 rpm at 20 hrs. and then increased to 800 rpm at 44.5 hr.

Fermentation times range from 60—70 hrs with beer viscosity ranging from 3500 to 4800 mPa · s. Conversion efficiencies vary from 44—55% with 3% glucose. The antifoam sold under the registered Trade

6

Mark Antifoam SAG 471" (Union Carbide) is used. A particular fermentation under these conditions lasted 69 hours.

4. Recovery

On completion of the fermentation, the heteropolysaccharide S-198 may be recovered by treatment of the fermentation beer with a miscible solvent which is a poor solvent for the heteropolysaccharide and does not react with it. In this way the heteropolysaccharide is precipitated from solution. The quantity of solvent employed generally ranges from about 2 to about 3 volumes per volume of fermentation beer. Among the various solvents which may be employed are acetone and lower alkanols such as methanol, ethanol, isopropanol, n-butanol, sec-butanol, tertiary butanol, isobutanol, and n-amyl alcohol. Isopropanol is preferred. Typically, the fermentation beer is heated to a temperature of about 70°C to 75°C for a short time (e.g., about 10 to 15 minutes), and then cooled to about 30°C or lower before addition of the solvent. A spent alcohol concentration of 57—59% is required for precipitating the heteropolysaccharide from the fermentation beer. The solid is recovered by separating it from the liquid, as by filtering or straining, and then drying at elevated temperature. This particular fermentation batch was pasteurized at 75°C for 15 minutes and then precipitated with approximately 2 volumes of isopropanol.

5. Drying

The product is dried at 55°C for up to one hour in a forced-air tray drier.

6. Product quality

Typical one percent deionized water viscosities range from 1200—1600 mPa · s as measured on a Brookfield LVF, spindle 3, 60 rpm at 25°C.

The results from one fermentation are given below.

| Age (hr.) | pH | Visc. (mPa · s) | Residual carbon source | Yield |
|---|---|---|---|---|
| 20 | 6.4 | 1300 | 1.98% | 0.53% |
| 44.5 | 6.5 | 2900 | 0.81% | 1.20% |
| 69 | 6.6 | 3650 | 0.30% | 1.46% |

Viscosities are measured on a Brookfield LVF viscometer at 60 rpm, room temperature, with spindle 2 (<500 mPa · s), 3 (500—2000 mPa · s), or 4 (>2000 mPa · s).

Heteropolysaccharide S-198

The heteropolysacchride produced by ATCC 31853 is composed of principally carbohydrate, 2—4% (calculated as O-acetyl) O-acyl groups, substantially no pyruvate, and about 9—12% protein.

The carbohydrate portion of the S-198 polysaccharide contains 13.0—17.6% glucuronic acid (based on wt. gum) and the neutral sugars mannose, glucose, and rhamnose in the approximate molar ratio of 1:2:2.

Colloidal titration (DIMDAC/sulphonic acid method) indicates the gum is acidic (0.58% equivalents of anionic groups/g. gum). Potentiometric titration indicates 0.65 mequiv./gm.

The acetyl content of 2—4% was determined by treating a 0.2% aqueous solution of S-198 gum with an alkaline, hydroxylamine reagent followed by treatment with an acidic ferric chloride reagent [S. Hestrin (1949) *J. Biol. Chem.* 180 249—261].

Liquid chromatography (Bio-Rad Organic Acid Analysis Column) was used to further identify the O-acyl groups. Using this technique the O-acyl groups of S-198 have been found to be primarily O-acetyl and O-succinyl, and substantially free of O-formyl.

The neutral sugars of polysaccharide S-198 were determined by dissolving ten mg. of the product in 2 ml 2N $H_2SO_4$, and the mixture is heated at 100°C for 4 hours. The resulting solution is cooled, neutralized with barium hydroxide and the pH is brought to 5—6 with solid carbon dioxide. The resulting precipitate of barium sulfate is removed by centrifugation and the supernatant is concentrated to a syrup under reduced pressure. The sugars in the hydrolysate are tentatively identified by gas-liquid chromatography of their aldononitrile acetate derivatives on a Hewlett-Packard Model 5830A chromatograph using 3% by weight OV-225 on 80/100 mesh Gas Chrom Q at 210°C. The sugars are identified and quantitated by comparison with authentic standards (J. K. Baird, M. J. Holroyde, and D. C. Ellwood (1973) Carbohydr. Res. 27 464—467]. See Table 2 for determination of neutral sugars.

**0 064 354**

TABLE 2
Total neutral sugars in S-198

| Sugar | Wt. % |
|---|---|
| Rhamnose | 38—42 |
| Glucose | 39—43 |
| Mannose | 16—19 |

The various neutral sugars of the polysaccharides were also characterized by use of descending paper chromatography on Whatman No. 1 ("Whatman" is a registered Trade Mark) chromatography paper using as the solvent the upper layer of pyridine:ethyl acetate:water (2:5:5). Chromatograms were stained using silver nitrate dip and acid aniline phthalate spray reagent. Component sugars were identified by co-chromatography with sugar standards and by the specific-color reaction with the aniline phthalate reagent.

The glucuronic acid content of the polysaccharide was determined by decarboxylation with 19% hydrochloric acid, followed by trapping the liberated carbon dioxide in standard sodium hydroxide and back titration [B. L. Browning (1967) *Methods of Wood Chemistry II*, 632—633]. The decarboxylation method gave the value 17.6, 13.1, and 14.9% for three different samples of S-198.

Paper electrophoresis was used for the separation and tentative identification of the uronic acids present in the neutralized acid hydrolysate described above. Portions of this and known uronic acid standards were applied to Camag electrophoresis paper No. 68—011 and electrophoresis was carried out for 2.0 hours in a pH 2.7 buffer using a Camag Model HVE electrophoresis apparatus. Chromatograms were air dried and stained with silver nitrate dip reagent to locate the uronic acids being separated. Two uronic acid spots were found by this methd which had the relative mobilities of glucuronic acid and mannuronic acid standards, the latter spot representing an acid resistant uronic acid-containing disaccharide.

An infrared spectrum of native S-198 was made on dried material in a KBr pellet. The heteropolysaccharide evidenced peaks at: 1725 $cm^{-1}$ and 1625 $cm^{-1}$ indicating ester and carboxyl groups, respectively.

A sample of S-198, BD-791, was dialyzed and freeze dried after fermentation. The sample was methylated according to the procedure used in Sanford and Conrad, Biochemistry 5 (1966) 1508—1517).

Approximatley 40 mg of sample was weighed into dry 100 ml serum bottles before inserting a rubber serum cap. Then *ca.* 40 ml of dimethyl sulfoxide (DMSO) (redistilled and dried over 4A molecular (sieves) was added via a syringe. The sample was continuously flushed with dry nitrogen. In order to dissolve the sample, it was necessary to heat the solution. To the vial was added 20 ml anion solution made from DMSO and NaH (2.5 g/50 ml DMSO). The sample was placed in sonic water bath for *ca.* 30 min before leaving at room temperature overnight. The solution was cooled and then 20 ml $CH_3I$ was added via a syringe. After stirring for at least one hour, the excess $CH_3I$ was rotovaped off before dialysis (DI water) and concentrating to dryness (rotovap).

The sample was hydrolyzed using 90% formic acid/0.13$M$ $H_2SO_4$. The resulting sugars were reduced with $NaBH_4$ and the resulting alditols were acetylated with acetic anhydride/pyridine (1:1) at 100°C.

The methyl sugars as their alditol acetates were separated by gas chromatography. Apparent identities were deduced by looking at both retention times (RT) and mass fragmentation patterns. The relative amounts of the methyl derivatives of glucose, mannose, and rhamnose are given in Table 3. Based on the amounts of the neutral sugars found (Table 2), it is believed that the 2,6-dimethyl sugar (Table 3) is 2,6-dimethyl glucose.

Fragments released by mild acid were also analyzed. A sample of S-198 was hydrolyzed under mild conditions (1.0$N$ $H_2SO_4$, 100°C) and the resulting fragments were separated by paper chromatography (pyridine:ethyl acetate:water (2:5:5), upper phase). Based on chromatograms developed with silver nitrate, S-198 was found to give spots corresponding in mobility to the reducing sugars rhamnose, mannose, glucose, glucuronic acid and a low proportion of an additional but unidentified fast moving component ($R_{Glc}$=3.30).

8

# 0 064 354

TABLE 3
Relative amounts of methyl sugars in S-198

| Methyl sugars | | Wt. % | Linkage | Approx. moles |
|---|---|---|---|---|
| 2,3,4 Me$_3$ | Rhamnose | 27.5 | Terminal | 2 |
| 2,6 Me$_2$ | Rhamnose | 14.2 | 1,4 | 1 |
| 2,4,6 Me$_3$ | Glucose | 13.3 | 1,3 | 1 |
| 2,3,6 Me$_3$ | Glucose | 4.1 | 1,4 | — |
| 2,3,6 Me$_3$ | Mannose | 21.7 | 1,4 | 1 |
| 2,6 Me$_2$ | Glu/Man | 19.3 | 1,3,4 | 1 |

Heteropolysaccharide S-198 has the following profile of properties (all measurements are at room temperature):

1. Viscosity (Brookfield LVT Viscometer)

| Conc. | Spindle | RPM | Tap H$_2$O | Viscosity (mPa · s) Tap H$_2$O +0.1% KCl |
|---|---|---|---|---|
| 1.0% | 4 | 60 | 2,450 | 2800 |
| — | 4 | 6 | 16,000 | — |
| 0.1% | 1+UL adap. | 6 | 76 | 75 |
| 0.5% | (Wells-Brookfield) | — | 590 | 560 |

2. Shear (Wells-Brookfield microviscometer RVT—mPa · s

1. n @ 1.92 sec$^{-1}$ 10,560 mPa · s     4. n @ 384 sec$^{-1}$ >64 mPa · s
2. n @ 9.6 sec$^{-1}$ 2,560 mPa · s     5. n @ 384 sec$^{-1}$ >64 mPa · s
3. n @ 76.8 sec$^{-1}$ 320 mPa · s     6. n @ 9.6 sec$^{-1}$ 1880 mPa · s

3. 4°C Storage stability (4 weeks)
Week 4: 2600 mPa · s, Brookfield LVT, spindle No. 4, 60 rpm; no gelation.

4. Acid, base, heat, stability

| A. Stability | Initial viscosity mPa · s | Final viscosity mPa · s | % Change | |
|---|---|---|---|---|
| 1. Acetic acid plus heat | 2700 | 2950 | +9 | Brookfield |
| 2. 1% HCl plus heat | 1040 | 1200 | +15 | " |
| 3. 1% NaOH plus heat | 680 | 2200[a] | +224 | Wells-Brookfield |
| 4. Heat only | 1410[b] | 1550[c] | +10 | " |

[a] gel
[b] 0.75% soln.
[c] softgel

B. pH Effect (Wells-Brookfield RVT—mPa · s @ 9.6 sec$^{-1}$)

| | | | |
|---|---|---|---|
| 1. 5% Acetic acid | 2.61 | pH | 2230 cP |
| 2. 5% NH$_4$OH | 11.15 | pH | 2560 cP |

5. Salt & dye compatibility
A. Salt

| | | | |
|---|---|---|---|
| 1. $CaCl_2$ (Saturated) | Compatible | 5. 1% $CaCl_2 \cdot 2H_2O$ | Compatible |
| 2. Amm. polyphosphate | Precipitate | 6. 1% KCl | Compatible |
| 3. 60% $NH_4NO_3$ | Compatible | 7. 0.1% KCl | 990 mPa $\cdot$ s[d] |
| 4. 1% $Al_2(SO_4)_3 \cdot 18H_2O$ | Compatible | 8. 2.5% KCl | 1380 mPa $\cdot$ s[d] |

[d] Wells-Brookfield RVT—mPa $\cdot$ s @ 9.6 sec$^{-1}$

B. Dyes

| | | | |
|---|---|---|---|
| 1. Milling Green | Compatible | 2. Methylene Blue | Precipitate |

6. Texture/flow properties
   Chunky, discontinuous flow, no gelation, not gummy to the touch.

7. Synergism & enzymes (Wells-Brookfield RVT—mPa $\cdot$ s at 9.6 sec$^{-1}$)

| | Viscosity 1% mPa $\cdot$ s | 0 hour viscosity in mPa $\cdot$ s of mixture | 2 hour viscosity in mPa $\cdot$ s of mixture | Expected viscosity mPa $\cdot$ s | Synergism |
|---|---|---|---|---|---|
| A Guar | 2050 | 1740 | 1920 | 1700 | +13% |
| B. H.P. Guar | 2230 | 1840 | 2020 | 1790 | +13% |
| C. Carboxymethylcellulose | 560 | 850 | 720 | 890 | None |
| D. Hydroxyethylcellulose | 440 | 1100 | 770 | 790 | None |

8. Milk reactivity
   A. Dispersion: Excellent
   B. Whey off: 1st day

9. Film formation
   Medium tensile strength, rough texture; uneven consistency; uneven pull down plastic film formed.

Cross-linking of S-198 with various polyvalent ions was tested by preparing 0.4% S-198 solutions in 2% KCl tap water. To 200 ml of these solutions were added, respectivley: 1) 1.0 gm chrome alum crystal dissolved in 5 ml $H_2O$; 2) 1.0 gm aluminum sulfate dissolved in 5 ml $H_2O$; 3) 1.5 ml Zircomplex® (Manchem, Manchester), liquid zirconium; 4) 1.5 ml Tysor LA® (Dupont), organic titanate; and 5) 0.5 gm $FeCl_3$ dissolved in 5 ml $H_2O$. These solutions were allowed to stand for 15 min., then checked for cross-linking. If no cross-linking was observed, the pH was raised to 9.0 using 15% NaOH and the solutions re-checked. Fresh solutions were prepared and the pH's adjusted with either 15% NaOH or 15% HCl to 4, 7, and 9 and checked for cross-linking after 15 min. S-198 did not cross-link under any of these conditions with $Cr^{+3}$ or $Al^{+3}$. It did cross-link with $Zr^{+4}$, $Ti^{+4}$, and $Fe^{+2/3}$ but only at pH 9.0 Cross-linking was evidenced by weak or moderate gelation.

The polysaccharide S-198 imparts viscosity to aqueous media when dissolved in water in low concentrations. It is useful as a thickening, suspending, emulsifying, stabilizing, lubricating, film-forming, or binding agent, especially in aqueous systems. In particular, it has uses in the following applications or products: adhesives, wall-joining cements, water-retentive grouts and mortars, spackling compounds, can scaling, boiler compounds, latex creaming, welding-rod fluxes, brazing pastes, ceramic glazes and extrusions, cleaners and polishes, toys, emulsions (latex, asphalt, silicone), silver recovery, seed coatings, spray control for pesticides or herbicides, emulsifiable concentrated and flowable pasticides and herbicides, tobacco binders, water-based inks, lithographic fountain solutions, leather finishes, hydromulching and hydro-seeding, textile printing and finishing, wet-end paper additives, wet-end paper retention and formation aid, anti-stick compounds, mold-release agents, liquid resins, slurry and packaged explosives, petroleum and water-well drilling muds, petroleum workover and completion fluids, petroleum stimulation fluids, cosmetics, pharmaceutical suspensions and emulsions.

Also this gum has utility in food systems such as jellies and other high sugar systems, beverages including citric acid based drinks, dairy products including ice cream and yogurt, salad dressings, dry mixes, icings, and glazes, syrups, puddings, farinaceous foods, canned and retorted foods, and bakery fillings.

In paper applications S-198 could be used, for example, in a pigmented clay coating of the following formulation:

| | |
|---|---|
| Pigment (Satin White) | 10% |
| Clay | 90% |
| S-198 | 0.5% |
| Latex | 10 pts/100 pts/pigment |
| Soy proteins | 5 pts/100 pts/pigment |

These ingredients are mixed with sufficient water to produce an easily applicable paper coating.
S-198 can be used in no-oil, low-oil, or high-oil salad dressings. Typical formulations are:

### No-oil dressing

| Ingredients | Percent |
|---|---|
| Water | 50.90 |
| Cider vinegar (50-grain) | 29.80 |
| High Fructose corn syrum | 10.00 |
| Lemon juice, single strength | 5.00 |
| Salt | 2.20 |
| Spices & Other ingredients | 1.40 |
| Sodium Citrate | 0.10 |
| S-198 | 0.325 |

### 6% Oil dressing

| Ingredients | Percent |
|---|---|
| $H_2O$ | 56.10 |
| Vinegar (50 grain) | 18.00 |
| Tomato paste (26%) | 7.50 |
| Vegetable oil | 6.00 |
| Lemon juice | 5.00 |
| Salt | 3.50 |
| Egg yolk (fresh) | 2.00 |
| S-198 | 0.45 |
| Spices and other ingredients | 1.3 |

38% oil dressing

| Ingredients | Percent |
|---|---|
| Vegetable oil | 38.00 |
| H₂O (tap) | 36.50 |
| Sugar (white, 100 grain) | 9.00 |
| Salt | 2.00 |
| Paprika | 1.35 |
| Mustard | 1.25 |
| S-198 | 0.40 |

Because of a tendency to gel, less than 0.4% S-198 is recommended in high oil salad dressings.

HWBF's comprise at least 90% water, the balance being various additives which vary with the specific use of the HWBF. The additives are, generally, wetting agents, defoamers, thickeners, liquid and vapor phase corrosion inhibitors, anti-wear agents, extreme pressure material, and biological control agents.

There are three major types of HWBF available:

1. Soluble Oil-in-Water Emulsion: minute droplets of oil suspended in water with emulsifying agents.
2. Synthetic Solution: 5% soluble salt additives in water, a true solution containing no oil.
3. Micro-emulsions: a combination of the soluble oil-in-water type and synthetic solution; the size of the oil droplet is in the range of 2 microns (or less).

Representative HWBF's commercially available are those sold under the registered Trade Marks:

| | |
|---|---|
| Hydrolubric 120 B | Synthetic solution |
| Hydra-cut 496 | Synthetic solution |
| Mobilmet 160 | Oil-emulsion |
| MTL 78 | Synthetic solution |
| Sunsol HWBF | Micro-emulsion |
| Sunsol AW | Oil-emulsion |
| Wyandotte B-1 | Synthetic solution |

Compounds such as cellulose, xanthan gum, and alginates are known as thickeners or viscosity modifiers. See e.g., GB 2,027,445.

HWBF's can be prepared which incorporate heteropolysaccharide S-198 as the sole viscosity modifier. HWBF's prepared with S-198 exhibit resistance to degradations under conditions of high temperature and high shear.

HWBF's prepared with S-198 are particularly useful as hydraulic fluids and lubricating fluids. Because of the danger of fire in certain applications, HWBF's containing no flammable components are particularly desirable.

As lubricating agents, S-198 HWBF's can be used in a broad spectrum of machinery requiring fluid lubrication.

Although the specific formulation may vary depending on the particular application, HWBF's of the following formulation are recommended:

12

**0 064 354**

| Ingredient | Wt. % |
|---|---|
| Water | 90—95 |
| Corrosion inhibitor | 0.1—2 |
| Anti-wear additive | 0.1—2 |
| Wetting Agent | 0.5—5 |
| Defoamer | 0.1—0.5 |
| Preservative | 0.1—0.5 |
| Viscosity modifier | 0.1—2 |

Optionally, where extremely low temperatures are expected, 10—33% ethylene glycol, or other antifreeze, may be added to improve freeze-thaw stability or pour point.

Typical corrosion inhibitors are: imidazoline, benzotriazole, sodium nitrile, morpholine, and triethanolamine. Anti-wear additives comprise sulphur-phosphorus containing compounds. These also function as extreme pressure additives when HWBF's are used in very high pressure systems. Wetting agents can be the salts of alkylaryl sulfonic acids and phosphate surfactants. The defoamer can be either of the silicone or non-silicone type. The preservative is a biological control agent, such as either one or a blend of a bactericide and a fungicide. The viscosity modifier is S-198.

S-198 shows excellent compatibility with either ethylene glycol or propylene glycol. At 0.5% concentration, an S-198 solution is pourable at 10°C in a 2:1 mixture of water:ethylene glycol. This property is advantageous over presently available HWBF's.

HWBF's prepared with S-198 can be used in a broad variety of pumps; e.g., piston, gear, and vane pumps.

Well treating fluids refers to a broad spectrum of media that are used to facilitate operations during the various stages of drilling and using a well, such as a gas or oil well. The term "well treating fluids" comprises, for example, circulating drilling fluids, workover and completion fluids, coring fluids, and stimulation fluids (hydraulic fracturing and acidizing). Materials which may be present in such fluids include sodium chloride, potassium chloride, barium sulfate, amorphous silica, calcium carbonate, bentonite, attapulgite, sodium metasulfate, quebracho, calcium lignosulfonate, lime, calcium sulfate, calcium chloride, petroleum sulfonate, tall oil soap, crude and diesel oils, starches, biocides, and polymers such as carboxymethylcellulose (CMC) polyacrylamides, and polyacrylates. It will be appreciated that not all of these compounds will be present in any particular fluid, but, rather, that compounds will be selected by the well operator in the amount necessary for the particular task to be performed. Because of the differing underground conditions experienced during and after a well is drilled, adjustments to the well treating fluid and replacement of one fluid with another are to be expected.

In addition to the compounds indicated above, the heteropolysaccharide xanthan gum is used extensively in many well treating fluids (see Can. Pat. 745,085).

Heteropolysaccharide S-198 is particularly useful in aqueous media especially formulated for use as a well-treating fluid.

S-198 has been found to exhibit properties such as pseudoplasticity, high working yield value, heat and salt stability, resistance to acid, shear stability, and good viscosity recovery after heating and cool-down which make it desirable as a rheology modifier in well-treating fluids.

Representative well-treating fluid formulations are as follows:

13

# 0 064 354

| | Ingredient | | |
|---|---|---|---|
| 1. | Seawater | 1 bbl | (159 litres) |
| | S-198 | 0.25 to 3.5 lb. | (113 g to 1.6 Kg) |
| | NaOH | to *ca.* pH 9 | |
| | Starch (optional) | 1 lb. | (454 g) |
| 2. | Fresh Water | 1 bbl | (159 litres) |
| | S-198 | 0.25 to 3.5 lb. | (113 g to 1.6 Kg) |
| | KCl | 3.5 to 17.5 lb. | (1.6 to 7.9 Kg) |
| | Bentonite | 2 to 20 lb. | (900 g to 9 Kg) |
| | NaOH | to *ca.* pH 9 | |
| | CMC (optional) | ½ lb. | (226 g) |
| 3. | Fresh Water | 1 bbl | (159 litres) |
| | S-198 | 0.25 to 3.5 lb. | (113 g to 1.6 Kg) |
| | Bentonite | 2 to 20 lb. | (900 g to 9 Kg) |
| 4. | Aq. HCl (1—15 wt. %) | 99% | |
| | Corrosion inhibitor | 0.05% | |
| | S-198 | 0.1—1.0% | |
| 5. | Fresh Water | 1 bbl | (159 litres) |
| | S-198 | 0.25—3.5 lb. | (113 to 1.6 Kg) |
| | KCl | 3.5—17.5 lb. | (1.6 to 7.9 Kg) |

Formulation 4 is useful as an acidizing fluid. The last formulation is suited for hydraulic fracturing and may also contain effective amounts of surfactants, breakers, cross-linking agents, and proppants (such as sand).

These formulations are not intended to be limiting but are suggestive of the range of possible well treating fluid formulations that can be prepared with S-198. For example, S-198 fermentation beer could be used as a well treating fluid.

S-198 is usable in such formulations in the range of 0.07—1% (wt.) or 0.25—3.5 lbs/bbl. A preferred range is 0.14—0.57%.

The invention also encompasses methods of treating wells which comprise introducing into a well aqueous media comprising water and 0.07—1% (wt.) S-198.

This invention also relates to the field of flowable pesticides, by which is meant pesticide formulations which present suspension stability problems because of the incorporation therein of insoluble, usually hydrophobic, active materials. Occasionally, the suspension problem is aggravated by the addition of inert diluents which are also insoluble, e.g. kaolin or other clays.

S-198 is particularly useful in preparing flowable pesticide compositions which contain insoluble or hydrophobic ingredients.

By pesticide is meant a broad spectrum of compounds that kill or inhibit the growth of animal and plant pests, thus, pesticides include insecticides, herbicides, fungicides, miticides, and aquaherbicides.

A common problem with these compounds is that many are either insoluble in water or hydrophobic. Typically, pesticide concentrates have to be subjected to large amounts of shear in order to prepare suspensions stable enough for storage until actually diluted and applied. S-198 retains its viscosity and high working yield value under conditions of high shear and thus is an excellent stabilizer for flowable pesticide concentrates of this sort.

In addition to active ingredient and suspension stabilizer, flowable pesticides also contain other ingredients such as antifreeze, surfactants, dispersants, and antifoams. A typical flowable pesticide formulation (concentrate) according to this invention is:

14

**0 064 354**

| Ingredient | %(wt.) |
|---|---|
| Water | 30—60 |
| Antifreeze | 3—10 |
| Surfactant | 1—3 |
| Dispersant | 1—4 |
| Antifoam | 0.25—1 |
| Active material | 10—60 |
| S-198 | 0.08—0.25 |

In final use, this concentrate is diluted to a final active ingredient concentration appropriate to the ingredient used and the nature of the infestation.

In the following illustrative Examples, "Morwet" "Igepal" Puraflow", "Orzan" and "Dowicil" are registered Trade Marks.

Example 1
Preparation of S-198
2. Seed preparation
ATCC 31853 was started in a 500 ml flask containing 100 ml YM medium incubated at 30°C. The YM seeds were then used at 24—30 hrs. to inoculate a 5L fermentor containing 3L of the seed medium. The composition of the seed medium is:

| | |
|---|---|
| 3.0% | Glucose |
| 0.5% | $K_2HPO_4$ |
| 0.05% | Promosoy 100® |
| 0.09% | $NH_4NO_3$ |
| 0.01% | $MgSO_4 \cdot 7H_2O$ |
| 2 ml | antifoam additive (SAG)® |

A 12% inoculum was used at 24 hrs. to inoculate the final fermentor, containing 20L of medium.

2. 30L Fermentor medium

| | |
|---|---|
| 3.0% | Glucose |
| 0.05% | $K_2HPO_4$ |
| 0.05% | Promosoy 100® |
| 0.09% | $NH_4NO_3$ |
| 0.01% | $MgSO_4 \cdot 7H_2O$ |
| 2 ml | antifoam additive (SAG)® |

The pH is controlled at 6.6—6.8; the temperature is 30°C. Aeration is 10L/min. Agitation is 300 rpm to start, increased to 700 rpm at 24 hr. Fermentation was stopped at 145 hr, giving a beer with a viscosity of 3000 mPa · s. The gum yield was 1.19%. Recovery of the polysaccharide was with 2—3 volumes of IPA. The solid was recovered and then dried at elevated temperature. The product was identified as BD-791. It exhibited a 1% viscosity of 1580 mPa · s.

15

Example 2

S-198 taxonomy

A. Morphological observations

1. Colonial morphology

In nutrient agar, small colonies appeared in two days, and the diameter of the colonies reached approximately 1.2 mm after three days' incubation. The colony was yellow pigmented (non-diffusible), round, smooth, entire, convex and opaque. A hard membranous film was formed, some colonies became umbonate after prolonged incubation.

On YM agar, small mucoid colonies appeared in two days, and the diameter of the colonies reached approximately 2.5 mm after three days' incubation. The colony was yellow pigmented (non-diffusible), round, smooth, entire, more drop-like and opaque. The texture of the colony became very membranous, and, entire colonies were often removed when they were pushed by inoculation needles.

2. Cellular morphology

The strain was a Gram-negative rod shaped bacterium, motile by peritrichous flagella. On nutrient agar, the size of the cell was 0.5—0.6 by 1.2—1.8 µm, the cell was straight and often had tapered ends. Elongation was well as pleomorphism were often seen after prolonged incubation.

On YM agar, the cell was straight, but more round-ended. The size of the cell was 0.4—0.6 by 0.9—2.4 µm. Pallisade arrangement of the cells was often seen in the Zooglea-like mass.

Capsule, spore, accumulation of poly-β-hydroxybutyrate and volutin granules were not observed. Acid fast stain was negative.

3. Growth in broth culture

Growth of the strain was limited only on the surface. Pellicle was formed in both nutrient and YM broths in test tube culture.

B. Physiological and biochemical tests

The following is a summary of results of physiological and biochemical tests employed which are listed in Table 4. Both cytochrome oxidase and catalase were positive. The organisms utilize glucose oxidatively, but not fermentatively. No anaerobic growth was observed. The organisms could grow at temperatures between 30 and 37°C, but not at 4° or 40°C. No survival was observed after incubation at 60°C for 30 minutes. Maximum concentration of NaCl for tolerance test was 1.5%. The organisms grew at both pH's 6 and 8, but not at pH's 4 and 10.

On TSI agar, growth was observed on the slant only, but neither acid nor alkali reaction was observed. Litmus milk was peptonized and acid was produced. No $H_2S$ was produced from either cystic broth and TSI agar. Ammonia was produced from peptone. The organism hydrolyzed starch, gelatin, esculin, Tween 80®, but not casein and pectin. 3-Ketolactose and Congo Red absorption tests were negative. The organisms were tolerant to 0.02 and 0.1% of triphenyltetrazolium chloride.

C. Nutritional tests

Growth factors were not required for their growth. Ammonium salt served as sole nitrogen source.

A total of 129 substrates were employed for nutritional tests, of which thirty (30) substrates were utilized by the organisms as carbon source and energy.

| | | |
|---|---|---|
| D-Xylose | Cellobiose | Sebacate |
| L-Arabinose | Lactose | L-Malate |
| L-Rhamnose | Inulin | DL-p-Hydroxybutyrate |
| D-Glucose | Gluconate | DL-Lactate |
| D-Mannose | Salicin | Meso-Inositol |
| D-Galactose | Acetate | p-Hydroxybenzoate |
| Fructose | Succinate | L-α-Alanine |
| Sucrose | Fumarate | DL-Isoleucine |
| Trehalose | Pimelate | L-Glutamate |
| Maltose | Agelate | L-Tyrosine |

The organism could not utilize alcohols and amines.

16

# 0 064 354

D. Antibiotic susceptibility tests

The organism was susceptible to chlortetracycline, 5 µg; gentamicin, 10 µg; kanamycin, 30 µg; neomycin, 30 µg; novobiocin, 30 µg; pencillin, 10 units; tetracycline, 30 µg. The organism was susceptible to the following antibiotics, but resistant colonies appeared. They were carbenicillin, 100 µg; erythromycin, 15 µg; and polymyxin B, 300 units. The organism was resistant to colistin, 10 µg, and streptomycin, 10 µg.

E. Identification

The strain S-198 was a Gram-negative, rod shaped bacterium, motile by peritrichous flagella. The organism was strictly aerobic, glucose was utilized oxidatively, but not fermentatively. According to Bergey's Manual of Determinative Bacteriology (the 8th edition, Edited by R. E. Buchanan and N. E. Gibbons, published by the Williams & Wilkins Company, Baltimore, 1974), this organism belongs to a member of the genus *Alcaligenes*. There were four species of the genus *Alcaligenes* listed in the manual, however, the strain S-198 does not belong to any of them. From this standpoint, it is concluded that the organism is a new species of the genus *Alcaligenes.*

TABLE 4
Results of physiological and biochemical tests of organism S-198

| Test | Results | Test | Results |
|---|---|---|---|
| Cytochrome oxidase | + | Growth at various NaCl concentrations: | |
| Catalase | + | 0.5% (W/V) | + |
| | | 1.5% | + |
| Oxidative and | | 3.0% | − |
| Fermentative | | 6.0% | − |
| (OF) Tests | Oxidative | 7.5% | − |
| | | 10.0% | − |
| Anaerobic growth | − | | |
| Acid production from various carbohydrates: | | Growth at various pH values: | |
| | | pH 4 | − |
| Adonitol | − | pH 6 | + |
| L-Arabinose | + | pH 8 | + |
| Dulcitol | + | pH 10 | − |
| Ethanol | − | pH 12 | − |
| Fructose | + | | |
| Galactose | + | | |
| D-Glucose | + | Indole Test | − |
| Inositol | − | Methyl Red (MR) Test | − |
| Inulin | − | Voges Proskauer (VP) Test | − |
| Lactose | − | Simmons' citrate Test | − |

TABLE 4 (continued)

| Test | Results | Test | Results |
|------|---------|------|---------|
| Acid production from various carbohydrates: | | | |
| Maltose | + | Nitrate reduction | − |
| Mannitol | − | Litmus Milk | − |
| | | Acid and Peptonized | |
| Mannose | + | | |
| Melibiose | + | | |
| α-Methylglucoside | − | Arginine dihydrolase (ADH) Test | − |
| Raffinose | + | Lysine decarboxylase (LDC) Test | − |
| Rhamnose | + | Ornithine decarboxylase (DC) Test | − |
| Salicin | − | Ammonia From Pentone | + |
| Sorbitol | − | Phenylaline deaminase (PAD) Test | − |
| Sucrose | + | $H_2S$ Production | − |
| Trehalose | − | Urease Test | − |
| D-Xylose | + | Phosphatase Test | − |
| | | Egg Yolk Test | − |
| Growth in TSI agar: | | | |
| Slant | No change | Starch hydrolysis | + |
| Butt | No growth | Gelatin Hydrolysis | + |
| Gas Production | − | Casein hydrolysis | − |
| $H_2S$ Production | − | Esculin hydrolysis | + |
| | | Tween 80 hydrolysis | + |
| Growth at various temperatures: | | Pectin hydrolysis | − |
| 4°C | − | | |
| 30°C | + | 3-Ketolactose production | − |
| 37°C | + | Congo Red Absorption | − |
| 40°C | − | | |
| 43°C | − | Growth with Triphenyltetrazolium Chloride: | |
| 45°C | − | 0.02% | − |
| 50°C | − | 0.10% | + |
| Survival at 60°C for 30 min | | | |

Example 3

Shear stability test protocol of S-198 HWBF

An 0.5% aqueous solution of S-198 is prepared with D.I. water at 50°C under agitation until the gum is dissolved. The viscosity is then adjusted to ca. 200 mPa · s (Brookfield LVT viscosity) with cold D.I. water.

A Sprague, piston-type air operated pump (Teledyne) is used to evaluate shear stability.

The procedure is to pump the gum solution through the pump at 2000 psi (13.8 MPa) (constant), cool the solution to r.t. (22.2°C), centrifuge to remove trapped foam, then measured viscosity on a Brookfield LVT at 60 rpm, spindle 2. This procedure is repeated more times at 2000 psi (13.8 MPa), then at increased pressures. The following data are obtained from three S-198 samples and from a 1:1:1 blend of the three.

TABLE 5
Sprague pump data

|  | Sample 1 | Sample 2 | Sample 3 | 1:1:1 Blend |
|---|---|---|---|---|
| Initial Visc. | 198 | 202 | 202 | 193 |
| Gum Conc. (%) | 0.277 | 0.19 | 0.25 | 0.28 |
| 2000 psi (13.8 MPa) |  |  |  |  |
| 1 | 270 | 291 | 292 | 208 |
| 2 | 248 | 262 | 266 | 232 |
| 3 | 228 | 250 | 259 | 222 |
| 4 | 213 | 215 | 265 | 215 |
| 5 | 210 | 226 | 241 | 198 |
| 6 | 190 | 212 | 233 | 193 |
| 3000 psi (20.7 MPa) | 258 | 258 | 298 | 235 |
| 4000 psi (27.6 MPa) | 247 | 235 | 282 | 229 |
| 5000 psi (34.5 MPa) | 198 | 238 | 241 | 222 |

Example 4
Salt stability of S-198
A 0.28% S-198 solution (1 lb/bbl) is prepared and tested in fresh water containing 3% (wt.) KCl on a Fann 35 viscometer. The following data are obtained.

|  | RPM | | | | | |
|---|---|---|---|---|---|---|
|  | 600 | 300 | 200 | 100 | 6 | 3 |
| Visc. (mPa · s) | 8.8 | 11.6 | 14.1 | 20.5 | 235 | 321 |

Example 5
Temperature response and stability
A 0.28% S-198 solution in fresh water containing 3% (wt.) KCl is prepared at pH 7. Measurements are taken on a Fann 50 viscometer at 58.8 rpm (100 sec$^{-1}$). Stability under these conditions is very good up to 325°F/163°C.

|  | Temp (°F/°C) | | | | | | |
|---|---|---|---|---|---|---|---|
|  | 80°/27° | 100°/38° | 150°/65.5° | 200°/93° | 250°/121° | 300°/149° | 325°/163° |
| Visc. (mPa · s) | 52.0 | 49.1 | 46.4 | 44.0 | 43.8 | 43.2 | 42.6 |
| %80°F Visc. | 100% | 94.4% | 89.7% | 84.6% | 84.2% | 83.1% | 81.9% |

Example 6
Acid stability
A 0.28% S-198 solution is prepared in 7.5% HCl. Viscosity measurements are taken over a 5 hr. period at 300 rpm and 3 rpm using a Fann 35 V—G meter at 80°F (26.6°C). The following data are obtained.

At 300 rpm

|  | | | Time (hr.) | | | |
| 0 | 0.5 | 1 | 2 | 3 | 4 | 5 |
| --- | --- | --- | --- | --- | --- | --- |
| 14.3 | 14.1 | 13.9 | 13.7 | 13.7 | 13.5 | 13.3 |

At 3 rpm

|  | | | Time (hr.) | | | |
| 0 | 0.5 | 1 | 2 | 3 | 4 | 5 |
| --- | --- | --- | --- | --- | --- | --- |
| 300 | 278 | 257 | 214 | 214 | 214 | 193 |

Example 7
Fungicide formulation

A fungicide formulation using technical sulfur as the active ingredient is prepared by first mixing together all of the following ingredients except for the final amount of antifoam. This mix is then wet ground in a Dyno Mill for about 10 minutes to a particle size of 2—5 microns. The remaining antifoam is added and slowly mixed for 20 minutes.

| Formulation | % by wt. |
| --- | --- |
| Water | 38.12 |
| Ethylene glycol | 5.0 |
| Non-ionic surfactant (Igepal CO-630®)[1] | 1.5 |
| Na naphthalene formaldehyde condensate (MORWET D-425)® | 0.5 |
| Na butyl naphthalene sulfonate (MORWET B)® | 2.5 |
| Silicone antifoam (Antifoam A)[2] | 0.05 |
| Technical Sulfur | 52.0 |
| S-198 | 0.18 |
| Antifoam A | 0.15 |

[1] GAF
[2] DOW

The suspension is stored at −18°C and then thawed once a week for 10 weeks for evaluation. The maximum separation is 1—2%. Viscosity is stable over the range 5900—6200 mPa · s on a Brookfield LVT at 3 rpm. pH varies from 6.5—6.6. The suspension is checked for evidence of agglomeration, thickening, lumps and sediment using both a probe and by pouring the suspension out of its container. On pour-out, the suspension is also evaluated for hang-up; i.e., sticking to the sides of the container. No undesirable problems are observed in any of these tests.

Similar tests are run on samples stored at 5°C, rm. temp., and 50°C over a two month period. Similar results are obtained, except that at elevated temperatures there is a tendency to gel after more than two months of storage.

Example 8
Herbicide formulation
Following the procedure of Example 7, an atrazine-based herbicide is prepared of the following formulations:

| Formulation | % by wt. |
| --- | --- |
| Water | 42.75 |
| Ethylene glycol | 7.0 |
| Non-ionic polyol surfactant (Puraflow E4®)[1] | 1.8 |
| Na Lignin sulfonate (Orzan LS®)[2] | 1.0 |
| Na Naphthalene formaldehyde condensate (MORWET D-425)® | 2.0 |
| Preservative (Dowicil 75®)[3] | 0.1 |
| Silicone antifoam (Antifoam A)[3] | 0.05 |
| Atrazine[4] Tech | 45.0 |
| S-198 | 0.15 |
| Antifoam A | 0.15 |

[1] BASF Wyandotte
[2] Crown Zellerbach Corp.
[3] DOW
[4] 2-chloro-4-ethylamino-6-isopropylamino-S-triazine

The following data are obtained after 6 months of storage.

|  | Init. | 2 mos. | 6 mos. |
| --- | --- | --- | --- |
| Visc.[1] | | | |
| 60 rpm | 460 | 500 | 550 |
| 12 rpm | 900 | 1000 | 1100 |
| 3 rpm | 2000 | 2500 | 2700 |
| Settling | NA | None | None |
| Separation | NA | None | 2% |
| Pour out | good | good | good |
| pH | 7.8 | 7.6 | 7.6 |

[1] Brookfield LVT, spindle No. 3

**Claims for the Contracting States BE CH DE FR GB IT LI LU NL SE**

1. Heteropolysaccharide S-198, which is principally carbohydrate, comprising 2—4% (calculated as O-acetyl) O-acyl groups, the O-acyl groups being primarily O-acetyl and O-succinyl and substantially free of O-formyl, 13—17.6% glucuronic acid, and the neutral sugars mannose, glucose, and rhamnose in the approximate molar ratio 1:2:2, in which the ratio of terminally linked rhamnose to 1,4 linked rhamnose is 2:1 and the mannose is principally 1,4 linked.

2. Heteropolysaccharide S-198 as claimed in Claim 1 prepared in a fermentation medium substantially free of $Ca^{++}$ ions.

3. A process for producing heteropolysaccharide S-198 which comprises growing the organism ATCC 31853 in an aqueous nutrient medium by the submerged aerobic fermentation of an assimilable carbohydrate source and recovering the heteropolysaccharide S-198.

4. A process as claimed in Claim 3 in which the nutrient medium is substantially free of Ca$^{++}$.

5. A biologically pure culture of an *Alcaligenes* microorganism, ATCC 31853.

6. A high-water-based fluid characterized in that 0.1—2% by weight of Heteropolysaccharide S-198 as claimed in Claim 1 is incorporated into it as the sole viscosity modifier.

7. A high-water-based fluid as claimed in Claim 6 comprising, by weight, 90—95% water, 0.1—2% corrosion inhibitor, 0.1—2% antiwear additive, 0.5—5% wetting agent, 0.1—0.5% defoamer, and 0.1—0.5% preservative, and, optionally, 10—33% antifreeze.

8. A drilling fluid comprising water and 0.07—1% by weight of Heteropolysaccharide S-198 as claimed in Claim 1.

9. A method of treating wells that comprises introducing into a well an aqueous medium comprising water and 0.07—1% by weight of Heterpolysaccharide S-198 as claimed in Claim 1.

10. A flowable pesticide composition comprising water, an insoluble or hydrophobic pesticide and 0.08—29% by weight of Heteropolysaccharide S-198 as claimed in Claim 1.

**Claims for the Contracting State AT**

1. A process for producing heteropolysaccharide S-198 which is principally carbohydrate, comprising 2—4% (calculated as O-acetyl) O-acyl groups, the O-acyl groups being primarily O-acetyl and O-succinyl and substantially free of O-formyl, 13—17.6% glucuronic acid, and the neutral sugars mannose, glucose, and rhamnose in the approximate molar ratio 1:2:2, in which the ratio of terminally linked rhamnose to 1,4 linked rhamnose is 2:1 and the mannose is principally 1,4 linked, that comprises growing the organism ATCC 31853 in an aqueous nutrient medium by the submerged aerobic fermentation of an assimilable carbohydrate source and recovering the heteropolysaccharide S-198.

2. A process as claimed in Claim 1 in which the nutrient medium is substantially free of Ca$^{++}$.

3. A high-water-based fluid comprising, by weight, 90—95% water, 0.1—2% corrosion inhibitor, 0.1—2% anti-wear additive, 0.5—5% wetting agent, 0.1—0.5% defoamer, and 0.1—0.5% preservative, and, optionally, 10—33% antifreeze, and characterised in that 0.1 to 2% by weight of Heteropolysaccharide S-198 is incorporated into it as the sole viscosity modifier.

4. A drilling fluid comprising water and conventional additives, characterised in that 0.07—1% by weight of Heteropolysaccharide S-198 is incorporated into it as the sole viscosity modifier.

5. A method of treating wells that comprises introducing into a well an aqueous medium comprising water and 0.07—1% by weight of Heteropolysaccharide S-198.

6. A method as claimed in Claim 5 in which the aqueous medium is a drilling fluid as claimed in Claim 4.

7. A flowable pesticide composition comprising water, an insoluble or hydrophobic pesticide, and 0.08—29% by weight of Heteropolysaccharide S-198.

**Patentansprüche für die Vertragsstaaten BE CH DE FR GB IT LI LU NL SE**

1. Heteropolysaccharid S-198, das hauptsächlich ein Kohlenhydrat ist, das 2—4% (berechnet als O-Acetyl) O-Acyl-gruppen, wobei die O-Acrylgruppen in erster Linie O-Acetyl und O-Succinyl und im wesentlichen frei von O-Formyl sind, 13—17,6% Glucuronsäure, und die neutralen Zucker Mannose, Glucose und Rhamnose in einem Molverhältnis von etwa 1:2:2 enthält, wobei das Verhältnis der endständig verknüpften Rhamnose zur 1,4-verknüpften Rhamnose 2:1 beträgt und die Mannose hauptsächlich 1,4 verknüpft ist.

2. Heteropolysaccharid S-198 nach Anspruch 1, hergestellt in einem Fermentationsmedium, das im wesentlichen frei von Ca$^{++}$ Ionen ist.

3. Verfarhen zur Herstellung des Heteropolysaccharid S-198, durch Züchten des Organismus ATCC 31853 in einem wässrigen Nährmedium, durch submerse aerobe Fermentation einer assimilierbaren Kohlenhydratquelle, und Gewinnen des Heteropolysaccharid S-198.

4. Verfahren nach Anspruch 3, bei dem das Nährmedium im wesentlichen frei von Ca$^{++}$ ist.

5. Biologisch reine Kultur eines Alcaligenes Mikroorganismus, ATCC 31853.

6. Ein stark auf Wasser basierendes Fluid, dadurch gekennzeichnet, daß 0,1—2 Gew.-% Heteropolysaccharid S-198, wie in Anspruch 1 beansprucht, als einziges die Viskosität modifizierendes Mittel eingearbeitet ist.

7. Ein stark auf Wasser basierendes Fluid nach Anspruch 6, enthaltend, bezogen auf das Gewicht, 90—95% Wasser, 0,1—2% Korrosionsinhibitor, 0,1—2% Antiverschleiß-Zusatz, 0,5—5% Benetzungmittel, 0,1—0,5% Entschäumunsmittel und 0,1—0,5% Konservierungsmittel, und gegebenenfalls 10—33% Antigefriermittel.

8. Bohrfluid, enthaltend Wasser und 0,07—1 Gew.-% Heteropolysaccharid S-198, wie in Anspruch 1 beansprucht.

9. Verfahren zur Behandlung von Bohrlöchern, durch Einführen in ein Bohrloch von einem wässrigen Medium, das Wasser und 0,07—1 Gew.-% Heteropolysaccharid S-198, wie in Anspruch 1 beansprucht, enthält.

10. Fließfähige Pestizidzusammensetzung, enthaltend Wasser, ein unlösliches oder hydrophobes Pestizid und 0,08—29 Gew.-% Heteropolysaccharid S-198, wie in Anspruch 1 beansprucht.

## Patentansprüche für den Vertragsstaat AT

1. Ein Verfahren zur Herstellung des Heteropolysaccharids S-198, welches hauptsächlich ein 2—4% (berechnet als O-Acetyl) O-Acylgruppen, wobei die O-Acylgruppen in erster Linie O-Acetyl und O-Succinyl darstellen, und im wesentlichen von O-Formyl frei sind, 13—17,6% Glucuronsäure, und die neutralen Zucker Mannose, Glucose und Rhamnose in dem angenäherten Molverhältnis 1:2:2, enthaltendes Kohlenhydrat derstellt, in welchem das Verhältnis von endständig gebundener Rhamnose zu 1,4-gebundener Rhamnose 2:1 beträgt und die Mannose hauptsächlich 1,4-gebunden ist, welches Verfahren das Züchten des Organismus ATCC 31853 in einem wässerigen Nährmedium durch die submerse aerobe Fermentation einer assimilierbaren Kohlenhydratquelle und die Gewinnung des Heteropolysaccharids S-198 umfaßt.

2. Ein Verfahren wie in Anspruch 1 beansprucht, in welchem das Nährmedium von Ca$^{++}$ im wesentlichen frei ist.

3. Eine Flüssigkeit mit hohem Wassergehalt, enthaltend 90—95 Gew.-% Wasser, 0,1—2 Gew.-% Korrosionsinhibitor, 0,1—2 Gew.-% Antiverschleißzusatz, 0,5—5 Gew.-% Netzmittel, 0,1—0,5 Gew.-% Entschäumungsmittel und 0,1—0,5 Gew.-% Konservierungsmittel, sowie gegebenenfalls 10—33 Gew.-% Gefrierschutzmittel, dadurch gekennzeichnet, daß in diese als einziges Viskositätsmodifikationsmittel 0,1 bis 2 Gew.-% Heteropolysaccharid S-198 einverleibt sind.

4. Eine Bohrflüssigkeit, enthaltend Wasser und übliche Zusätze, dadurch gekennzeichnet, daß in diese als einziges Viskositätsmodifikationsmittel 0,07—1 Gew.-% Heteropolysaccharid S-198 einverleibt sind.

5. Ein Verfahren zur Behandlung von Bohrlöchern, umfassend das Einführen eines wässerigen, Wasser und 0,07—1 Gew.-% Heteropolysaccharid S-198 enthaltenden Mediums in ein Bohrloch.

6. Ein Verfahren wie in Anspruch 5 beansprucht, bei welchem das wässerige Medium eine Bohrflüssigkeit ist, wie sie in Anspruch 4 beansprucht ist.

7. Eine fließfähige, pestizide Zusammensetzung, enthaltend Wasser, ein unlösliches oder hydrophobes Pestizid und 0,08—29 Gew.-% Heteropolysaccharid S-198.

## Revendications pour les Etats contractants BE CH DE FR GB IT LI LU NL SE

1. Hétéropolysaccharide S-198 qui est principalement un glucide, comprenant 2—4% de groupes acyles (calculés en O-acétyle), les groupes O-acyles étant principalement O-acétyles et O-succinyles et essentiellement dépourvus d'O-formyle, 13—17,6% d'acide glucuronique, et les sucres neutres mannose, glucose et rhamnose, dans le rapport molaire approximatif de 1/2/2, dans lequel le rapport du rhamnose à liaison terminale au rhamnose à liaison 1,4 est de 2/1, et le mannose est principalement à liaison 1,4.

2. Hétéropolysaccharide S-198 comme revendiqué dans la revendication 1, préparé dans un milieu de fermentation essentiellement dépourvu d'ions Ca$^{++}$.

3. Un procédé pour la production d'hétéropolysaccharide S-198 qui comprend la culture de l'organisme ATCC 31853 dans un milieu nutritif aqueux par fermentation aérobie submergée d'une source assimilable de glucide et le récupération de l'hétéropolysaccharide S-198.

4. Un procédé comme revendiqué dans la revendication 3, dans lequel le milieu nutritif est essentiellement dépourvu de Ca$^{++}$.

5. Une culture biologiquement pure d'un micro-organisme *Alcaligenes*, ATCC 31853.

6. Un fluide aqueux à forte teneur en eau caractérisé en ce que 0,1 à 2% d'hétéropolysaccharide S-198, comme revendiqué dans la revendication 1, lui sont incorporés comme unique modificateur de la viscosité.

7. Un fluide aqueux à forte teneur en eau comme revendiqué dans la revendication 6, comprenant, en poids, 90—95% d'eau, 0,1—2% d'inhibiteur de la corrosion, 0,1—2% d'additif antiusure, 0,5—5% d'agent mouillant, 0,1—0,5% d'antimousse et 0,1—0,5% de conservateur et éventuellement 10—33% d'antigel.

8. Un fluide de forage comprenant de l'eau et 0,07 à 1% en poids d'hétéropolysaccharide S-198 comme revendiqué dans la revendication 1.

9. Un procédé de traitement des puits qui comprend l'introduction dans un puits d'un milieu aqueux comprenant de l'eau et 0,07 à 1% en poids d'hétéropolysaccharide S-198 comme revendiqué dans la revendication 1.

10. Une composition pesticide fluide comprenant de l'eau, un pesticide insoluble en hydrophobe et 0,08 à 29% en poids d'hétéropolysaccharide S-198 comme revendiqué dans la revendication 1.

## Revendications pour l'Etat contractant AT

1. Un procédé de production d'hétéropolysaccharide S-198 qui est principalement un glucide, comprenant 2—4% de groupes acyles (calculés en O-acétyle), les groupes O-acyles étant principalement O-acétyles et O-succinyles et essentiellement dépourvus d'O-formyle, 13—17,6% d'acide glucuronique, et les sucres neutres mannose, glucose et rhamnose, dans le rapport molaire approximatif de 1/2/2, dans lequel le rapport du rhamnose à liaison terminale au rhamnose à liaison 1,4 est de 2/1, et le mannose est principalement en liaison 1,4, qui comprend la culture de l'organisme ATCC 31853 dans un milieu nutritif aqueux par fermentation aérobie submergée d'une source de glucide assimilable et la récupération de l'hétéropolysaccharide S-198.

2. Un procédé comme revendiqué dans la revendication 1, dans lequel le milieu nutritif est essentiellement dépourvu de $Ca^{++}$.

3. Un fluid aqueux à forte teneur en eau comprenant, en poids, 90—95% d'eau, 0,1—2% d'inhibiteur de la corrosion, 0,1—2% d'additif antiusure, 0,5—5% d'agent mouillant, 0,1—0,5% d'antimousse et 0,1—0,5% de conservateur et éventuellement 10 à 33% d'antigel, et caractérisé en ce que 0,1 à 2% en poids d'hétéropolysaccharide S-198 lui sont incorporés comme unique modificateur de la viscosité.

4. Un fluide de forage comprenant de l'eau et des additifs classiques, caractérisé en ce que 0,07—1% en poids d'hétéropolysaccharide S-198 lui est incorporé comme unique modificateur de la viscosité.

5. Un procédé de traitement des puits qui comprend l'introduction dans un puits d'un milieu aqueux comprenant de l'eau et 0,07 à 1% en poids d'hétéropolysaccharide S-198.

6. Un procédé comme revendiqué dans la revendication 5, dans lequel le milieu aqueux est un fluide de forage comme revendiqué dans la revendication 4.

7. Une composition pesticide fluide comprenant de l'eau, un pesticide insoluble ou hydrophobe et 0,08 à 29% en poids d'hétéropolysaccharide S-198.